(19) Europäisches Patentamt
European Patent Office
Office européen des brevets

(11) Veröffentlichungsnummer: **0 224 812 B1**

# (12) EUROPÄISCHE PATENTSCHRIFT

(45) Veröffentlichungstag der Patentschrift: **18.04.90**

(51) Int. Cl.⁴: **C07D 277/80**, C02F 1/26

(21) Anmeldenummer: **86116126.3**

(22) Anmeldetag: **21.11.86**

## (54) Verfahren zur Aufarbeitung von Mutterlaugen aus der Herstellung von benzthiazol-Verbindungen.

(30) Priorität: **04.12.85 DE 3542795**
**21.06.86 DE 3620822**

(43) Veröffentlichungstag der Anmeldung: **10.06.87 Patentblatt 87/24**

(45) Bekanntmachung des Hinweises auf die Patenterteilung: **18.04.90 Patentblatt 90/16**

(84) Benannte Vertragsstaaten: **BE DE FR GB IT NL**

(56) Entgegenhaltungen: **DE-A-1 940 365**

(73) Patentinhaber: **BAYER ANTWERPEN N.V., Kanaaldok B 1 Kruisschans, B-2040 Antwerpen 4(BE)**

(72) Erfinder: **Denecker, Gabriel, Dr., Vinkenlaan 18, B-2180 Kalmthout(BE)**
Erfinder: **Sluyts, Domien, Elzenlaan 4, B-2091 Stabroek(BE)**
Erfinder: **Biot, Jean-Marie, Dr., C/o Bayer do Brasil S.A. Cep 20 001, Rio de Janeiro R.J. C.P. 1500E650(BR)**
Erfinder: **van Osselaer, Tony, Dr., Tuinlaan 62, B-9180 Belsele(BE)**
Erfinder: **de Roos, Jan, Paul van Ostayenstraat 1, B-2750 Beveren (Waas)(BE)**
Erfinder: **Bamelis, Pol, Dr., Im Holz 20, D-5060 Bergisch Gladbach 3(DE)**

(74) Vertreter: **Gremm, Joachim, Dr. et al, Bayer AG Konzernverwaltung RP Patente Konzern, D-5090 Leverkusen 1, Bayerwerk(DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Aufarbeitung von wäßrigen Mutterlaugen, die aus der oxidativen Herstellung von Benzthiazolyl-sulfenamiden aus gegebenenfalls kernsubstituierten 2-Benzothiazolyldisulfiden, 2-Mercaptobenzothiazolen oder 2-Mercaptobenzothiazolsalzen und den entsprechenden Aminen sowie gegebenenfalls Schwefel stammen und nicht umgesetztes Amin oder einen als Lösungsvermittler eingesetzten, niedrig siedenden Alkohol oder beides enthalten.

Solche Mutterlaugen fallen bei der Herstellung von Verbindungen an, die als Vulkanisationsbeschleuniger verwendet werden. Beispiele sind:

Benzothiazolyl-2-cyclohexylsulfenamid, Benzothiazolyl-2-tert.butylsulfenamid, Benzothiazolyl-2-sulfenmorpholid, Benzothiazolyl-2-diäthylsulfenamid, Benzothiazolyl-2-diisopropylsulfenamid, Benzothiazolyl-2-dicyclohexylsulfenamid und 2-(4-Morpholinyldithio)-benzothiazol.

Aus der US-A-2 809 202 ist ein Verfahren zur Herstellung von Benzthiazolyl-sulfenamiden bekannt, wonach man die Mischungen aus wäßriger und organischer Phase trennt, die wäßrige Phase extrahiert und aus den vereinigten organischen Phasen die extrahierten Komponenten isoliert.

Diese Mutterlaugen enthalten weiterhin nicht vernachlässigbare Konzentrationen an schwefel- und/oder stickstoffhaltigen Nebenprodukten. Üblicherweise werden die Mutterlaugen durch destillative Rückgewinnung des organischen Lösungsmittels bzw. des Amins aufgearbeitet. Hierbei trennen sich schwerflüchtige, wasserunlösliche organische Nebenprodukte in Form von harzartigen Flocken bzw. Teer ab. Diese führen zu Verschmutzungen und Verstopfungen der Destillationsapparatur und der nachgeschalteten Anlageteile und machen zeitraubende und kostspielige Reinigungsarbeiten notwendig. Die übrigen, wasserlöslichen organischen Nebenprodukte, z.B. Benzothiazol-2-sulfinsäure und Benzothiazol-2-sulfonsäure und deren Salze bleiben in gelöster Form in dem so behandelten Abwasser zurück. Da bisher keine ökonomisch arbeitende Methode bekannt ist, diese Produkte aus dem Abwasser zu entfernen, besteht nur die Möglichkeit, nach teilweisem oder vollständigem Eindampfen die Rückstände zu verbrennen. Die Mutterlaugenaufarbeitung des genannten Verfahrens ist daher nicht nur mit verfahrenstechnischen Problemen behaftet, sondern auch sehr energieintensiv.

Aufgabe der Erfindung ist die Bereitstellung eines Aufarbeitungsverfahrens, mit dem die organischen Bestandteile aus der Mutterlauge entfernt werden, keine Verschmutzung oder Verstopfung durch Harze und Teer auftreten und ein Abwasser zurückbleibt, das, ohne verdampft und verbrannt werden zu müssen, an die Umwelt abgegeben werden kann.

Gegenstand der Erfindung ist ein Verfahren zur Aufarbeitung von wäßrigen Mutterlaugen, die aus der oxidativen Herstellung von Verbindungen der Formel

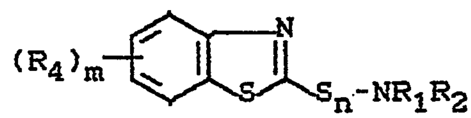

worin

m 0, 1 oder 2,

n 1 oder 2,

$R_1$ Wasserstoff, $C_1$- bis $C_6$-Alkyl oder $C_5$- bis $C_8$-Cycloalkyl,

$R_2$ $C_1$- bis $C_6$-Alkyl oder $C_5$- bis $C_8$-Cycloalkyl oder

$R_1$ und $R_2$ gemeinsam $-(CH_2)_5-$ oder

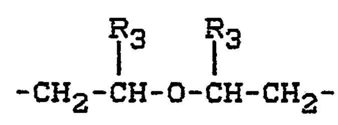

und

$R_3$ $C_1$–$C_6$-Alkyl und/oder Wasserstoff und

$R_4$ Wasserstoff oder $C_1$–$C_6$-Alkyl bedeuten

aus gegebenenfalls kernsubstituierten 2-Benzothiazolyldisulfiden, 2-Mercaptobenzothiazolen, 2-Mercaptobenzothiazolsalzen und den entsprechenden Aminen sowie gegebenenfalls Schwefel stammen und nicht umgesetztes Amin oder einen als Lösungsvermittler eingesetzten, niedrig siedenden Alkohol oder beides enthalten, dadurch gekennzeichnet, daß man die Mutterlauge durch Zusatz von Natriumsulfat und/oder Natriumchlorid in der Weise sättigt, daß man die pH-Werte mittels konzentrierter Schwefelsäure und/oder konzentrierter Salzsäure auf unter 2, vorzugsweise unter 1 einstellt, diese Mischung dann 3 Minuten bis 20 Stunden, vorzugsweise 30 Minuten bis 2 Stunden, bei 20 bis 250°C, vorzugsweise bei 60 bis 120°C, hält und dann den pH-Wert durch Zusatz konzentrierter Natronlauge auf 1 bis 11, vorzugsweise 4 bis 10, einstellt, wobei sich zwei Phasen bilden, die Phasen voneinander trennt, die wäßrige Phase mit weiterem Alkohol oder Amin bei 60 bis 120°C extrahiert, aus den vereinigten organischen Phasen das Extraktionsmittel und die extrahierten Komponenten isoliert und aus der wäßrigen Phase darin gelöstes Amin oder darin gelösten Alkohol abdestilliert.

Die in der organischen Phase extrahierten organischen und anorganischen Bestandteile erhält man nach Abdestillieren der leicht flüchtigen Alkohole und Amine in Form einer Schmelze, die wegen des hohen Aschegehaltes nicht ohne Entsalzung verwertet werden kann. In weiterer Ausgestaltung des erfindungsgemäßen Verfahrens wird daher bei der destillativen Rückgewinnung des Extraktionsmittels die organische Phase zunächst nur soweit aufkonzentriert, daß sich der Rückstand in zwei flüssige Phasen trennt, ein salzarmes Konzentrat von extrahierten Benzothiazolderivaten in Form eine Schmelze einerseits und eine wäßrige Lösung der mit extrahierten anorganischen Salze andererseits. Durch Trennung der beiden Phasen können organische und anorganische Produkte leicht voneinander getrennt und getrennt aufgearbeitet werden.

Die Schmelze kann auf verwertbare Substanzen aufgearbeitet oder zur Nutzung ihres Energieinhaltes verbrannt werden. Die wäßrige Lösung kann, gegebenenfalls nach Ausrühren mit Amin oder Alkohol in eine frühere Prozeßstufe wieder eingeschleust werden.

Enthält die aufzuarbeitende Mutterlauge nicht vernachlässigbare Restkonzentrationen an N-Chloraminen, so ist es zweckmäßig, diese vor der eigentlichen Aufarbeitung durch Zugabe von Reduktionsmitteln in üblicher Weise zu vernichten.

Für die Sättigung der aufzuarbeitenden Mutterlauge gibt es mehrere Verfahrensweisen, die auf jeden Fall so gestaltet werden müssen, daß es trotz der Sättigung nicht gleichzeitig zu einer Abscheidung von harzartigen Flocken oder Teer kommt.

So kann die wäßrige Mutterlauge der Synthesereaktion mit einer dazu ausreichend großen Menge Alkohol oder Amin vermischt werden. Diese Prozedur erfordert meistens verhältnismäßig große Mengen Alkohol oder Amin.

Das erfindungsgemäße Verfahren hat den Vorteil, daß polare Benzothiazolverbindungen, die sich in der Mutterlauge befinden, zu weniger polaren Verbindungen umgesetzt werden, die damit leichter in die organische Phase aufgenommen werden.

Die Trennung des Zweiphasensystems kann in jeder dazu geeigneten Trennapparatur durchgeführt werden. Ebenso besteht hinsichtlich der Extraktion der wäßrigen Phase durch weiteren Alkohol und/oder Amin hinsichtlich der Apparate keine Beschränkung. Vorteilhaft werden Gegenstromkolonnen oder gegebenenfalls mehrstufige Mischer/Abscheider-Kombinationen eingesetzt. Die für eine ausreichende Reinigung der wäßrigen Phase notwendige Zahl an theoretischen Extraktionsstufen beträgt etwa 3 bis 8.

Technisch elegant wird diese Extraktion mit Hilfe einer Kolonne durchgeführt, wobei die gesättigte Mutterlauge auf die Kolonne gegeben wird und sich dort trennt. Die wäßrige Phase fließt nach unten durch die Kolonne, Extraktionsflüssigkeit strömt, unten in die Kolonne eingespeist, der wäßrigen Phase entgegen und vereinigt sich an der Phasengrenzfläche am Kopf der Kolonne mit der organische Phase aus der Mutterlauge. Die vereinigten organischen Phasen werden über Kopf abgezogen und destillativ aufgearbeitet.

Die Aufkonzentrierung der organischen Phase kann ohne nennenswerte Anbackung oder Verschmutzung in allen für mittelviskose Flüssigkeiten geeigneten Verdampfern durchgeführt werden. Besonders geeignet hierzu sind unter anderem Fallfilmverdampfer, Dünnschichtverdampfer und Mehrphasenwendelrohrverdampfer. Ebenso kann auch der Alkohol und/oder das Amin mit konventionellen Destilliertechniken aus den Brüden der partiellen Aufkonzentrierung und aus der extrahierten wäßrigen Phase vollständig zurückgewonnen werden, ohne daß es zu nennenswerten Verschmutzungen der Destillationsapparatur und der nachgeschalteten Anlageteile kommt.

Bevorzugt wird die Destillation so betrieben, daß das zurückgewonnene Extraktionsmittel nicht mehr als 40 Gew.-% gelöstes Wasser enthält.

Pro Gewichtsteil der zu extrahierenden wässrigen Lösung werden dann 0,05–1, bevorzugt 0,1–0,5 Gewichtsteile Extraktionsmittel eingesetzt.

Beispiel 1 (Vergleichsversuch)

Zum Vergleich mit der Aufarbeitung von Mutterlaugen nach dem erfindungsgemäßen Verfahren (Beispiele 2 und 3) wird an Hand des nachfolgenden Beispiels der Stand der Technik illustriert.

35,2 kg einer Mutterlauge aus 23,2 Gew.-% Isopropanol, 0,86 Gew.-% organischem Kohlenstoff (exklusive Isopropanol) und 11,5 Gew.-% NaCl + $Na_2SO_4$, Rest Wasser aus der Herstellung von Benzthiazolyl-2-dicyclohexyl-sulfenamid werden zusammen mit 12 kg Waschwasser der Filtrationsstufe (31,7 gew.-%ig in Isopropanol) zur Rückgewinnung des Isopropanols aufdestilliert.

Die wasserunlöslichen Inhaltsstoffe der eingesetzten Mutterlauge trennen sich hierbei in Form einer teerartigen, viskosen Flüssigkeit ab, mit der Folge, daß die Destillationskolonne und die nachgeschalteten Anlageteile sehr oft gereinigt werden müssen. Als wäßrige Sumpfphase werden 33,2 kg eines stark verunreinigten Abwassers erhalten, aus dem durch Ausrühren mit verhältnismäßig großen Mengen Aktivkohle nur ein Bruchteil der organischen Kohlenstoffbelastung entfernt werden kann:

| | ohne Nachbehandlung | Nachbehandlung mit 10 g Aktivkohle pro Liter |
|---|---|---|
| Org. Kohlenstoff (Gew.-%) | 0,46 | 0,24 |
| CSB (mg 0/1) | 13 000 | 8300 |

Beispiel 2

35,2 kg einer Mutterlauge aus 22,8 Gew.-% Isopropanol, 0,98 Gew.-% organischem Kohlenstoff (exklusive Isopropanol) und 9,4 Gew.-% NaCl + $Na_2SO_4$, Rest Wasser aus der Herstellung von Benzthiazol-2-dicyclohexylsulfenamid (Ablauf Filteraggregat ; ohne Waschwässer), werden mit konzentrierter Schwefelsäure auf pH 0,5-0,8 gebracht, eine Stunde bei 80° C gerührt und unter Zugabe von einem Gemisch von 3,3 kg wäßriger Salzlösung aus der partiellen Aufkonzentrierung und von 1 kg Isopropanol-Destillat mit konzentrierter Natronlauge auf pH 7 bis 8 eingestellt.

Anschließend wird das hierbei gebildete Zweiphasengemisch in einer Füllkörperkolonne bei 70° C im Gegenstrom mit 4,8 kg Isopropanol-Destillat in Kontakt gebracht. Man erhält 23,5 kg extrahierte wäßrige Lösung I und 24,4 kg Extraktphase II, welche die nachfolgenden Analysewerte aufweisen:

| | Wäßrige Phase I | Organische Phase II |
|---|---|---|
| Isopropanol | 7,5 Gew.-% | 46,2 Gew.-% |
| Organ. Kohlenstoff (exkl. Isopropanol) | 0,095 Gew.-% | 1,57 Gew.-% |

Die Ausbeute der extraktiven Reinigung beträgt somit 94 %.

Durch partielles Aufkonzentrieren der isopropanolischen Phase II mit nachgeschalteter warmer Trennung des zweiphasigen flüssigen Rückstandes werden 640 g einer teerartigen Schmelze neben 3,3 kg einer wäßrigen Salzlösung isoliert, wobei die Salzlösung zur weiteren Reinigung in die Neutralisation zurückgeführt wird.

Schließlich werden 16,6 kg wäßriges Isopropanol (azeotrope Mischung) als Destillat aus den Brüden der partiellen Auskonzentrierung sowie aus der extrahierten wäßrigen Phase I und aus den verdünnten Waschwässern der Filtrationsstufe zurückgewonnen. Hierbei resultiert ein wasserklares und schmierenfreies Abwasser, das ohne Verschmutzung der Destillationsapparatur und der nachgeschalteten Leitungssysteme abgeführt wird.

Beispiel 3

Zu 20,2 kg einer Mischung von Mutterlauge und Waschwässern mit 3,0 Gew.-% Diisopropylamin (DIPA) und 0,3 Gew.-% organischem Kohlenstoff (exkl. DIPA) aus der Synthese von Benzthiazolyl-3-diisopropylsulfenamid wird bei 60° C Natriumsulfit gegeben, wobei sich 470 g einer DIPA-reichen Phase I abtrennen. Die hierbei erhaltene wäßrige Lösung wird mit konzentrierter Schwefelsäure auf pH 0,5 gestellt, über etwa eine Stunde bei 75° C gerührt und anschließend mit konzentrierter Natronlauge auf pH 8,0 gebracht, um dann in einer Füllkörperkolonne bei 60° C mit 5,4 kg 91 %igem DIPA extrahiert zu werden. Es resultieren einerseits 23,6 kg extrahierte wäßrige Phase II und andererseits 4,75 kg eines DIPA-reichen Extrakts III.

Die vereinigten organischen Phasen I und III werden zur Abtrennung der extrahierten Komponenten nach Zugabe von 260 g Wasser partiell aufkonzentriert, wobei ein Zweiphasengemisch anfällt, das sich durch warme Phasentrennung in einerseits 80 g einer praktisch salzfreien teerartigen Schmelze und andererseits 90 g einer wäßrigen Lösung aufteilen läßt.

Schließlich wird das Diisopropylamin aus den Brüden der partiellen Aufkonzentrierung sowie aus der extrahierten wäßrigen Phase II durch Destillation in azeotroper Konzentration zurückgewonnen, wobei 24 kg eines wasserklaren und schmierenfreien Abwassers mit 0,038 Gew.-% organischem Kohlenstoff (CSB: 1630 mg 0/1) erhalten werden.

Die Gesamtausbeute der Reinigungsprozedur beträgt 85 %.

**Patentansprüche**

1. Verfahren zur Aufarbeitung von wäßrigen Mutterlaugen, die aus der oxidativen Herstellung von Verbindungen der Formel

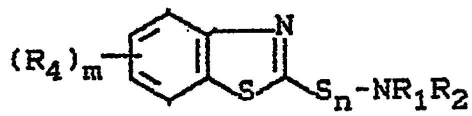

worin
m 0, 1 oder 2,
n 1 oder 2,
$R_1$ Wasserstoff, $C_1$- bis $C_6$-Alkyl oder $C_5$- bis $C_8$-Cycloalkyl,
$R_2$ $C_1$- bis $C_6$-Alkyl oder $C_5$- bis $C_8$-Cycloalkyl oder
$R_1$ und $R_2$ gemeinsam $-(CH_2)_5-$ oder

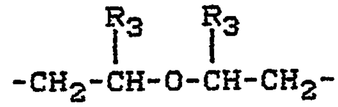

und
$R_3$ $C_1$–$C_6$-Alkyl und/oder Wasserstoff und
$R_4$ Wasserstoff oder $C_1$–$C_6$-Alkyl bedeuten,
aus gegebenenfalls kernsubstituierten 2-Benzothiazolyldisulfiden, 2-Mercaptobenzothiazolen oder 2-Mercaptobenzothiazolsalzen und den entsprechenden Aminen sowie gegebenenfalls Schwefel stammen und nicht umgesetztes Amin oder einen als Lösevermittler eingesetzten niedrig siedenden Alkohol oder beides enthalten, dadurch gekennzeichnet, daß man die Mutterlauge durch Zusatz von Natriumsulfat und/oder Natriumchlorid in der Weise sättigt, daß man den pH-Wert mittels konzentrierter Schwefelsäure und/oder konzentrierter Salzsäure auf unter 2 einstellt, diese Mischung dann 3 Minuten bis 20 Stunden bei 20 bis 250°C hält und dann den pH-Wert durch Zusatz konzentrierter Natronlauge auf 1 bis 11 einstellt, wobei sich zwei Phasen bilden, die Phasen voneinander trennt, die wäßrige Phase mit weiterem Alkohol oder Amin bei 60 bis 120°C extrahiert, aus den vereinigten organischen Phasen das Extraktionsmittel und die extrahierten Komponenten isoliert und aus der wäßrigen Phase darin gelöstes Amin oder darin gelösten Alkohol abdestilliert.

2. Verfahren nach Anspruch 1, dadurch gekennzeichnet, daß man die vereinigten organischen Phasen zur Rückgewinnung des Extraktionsmittels nur soweit aufkonzentriert, daß sich der Rückstand in zwei flüssige Phasen trennt, ein salzarmes Konzentrat von extrahierten Benzothiazolderivaten in Form einer Schmelze einerseits und eine wäßrige Lösung der mitextrahierten anorganischen Salze andererseits, die beiden Phasen voneinander trennt und getrennt aufarbeitet.

3. Verfahren nach den Ansprüchen 1 und 2, dadurch gekennzeichnet, daß man vor der eigentlichen Aufarbeitung in der Mutterlauge gegebenenfalls vorhandene N-Chloramine durch Zugabe von Reduktionsmitteln vernichtet.

4. Verfahren nach Ansprüchen 1 bis 3, dadurch gekennzeichnet, daß man mit konzentrierter Säure auf einen pH-Wert unter 1 und anschließend mit Natronlauge auf einen pH-Wert von 4 bis 10 einstellt.

5. Verfahren nach Ansprüchen 1 bis 4, dadurch gekennzeichnet, daß man die Mischung zwischen der Zugabe von Säure und Natronlauge 30 Minuten bis 2 Stunden bei 60 bis 120°C verweilen läßt.

**Claims**

1. A process for working up aqueous mother liquors from the oxidative production of compounds corresponding to the following formula

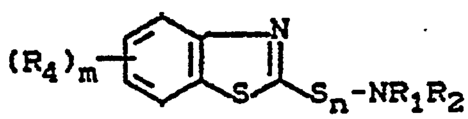

in which
m is 0,1 or 2,
n is 1 or 2,
$R_1$ is hydrogen, $C_{1-6}$ alkyl or $C_{5-8}$ cycloalkyl,
$R_2$ is $C_{1-6}$ alkyl or $C_{5-8}$ cycloalkyl or
$R_1$ and $R_2$ together represent $-(CH_2)_5-$ or

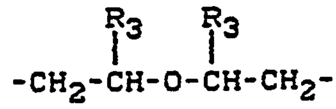

and
$R_3$ is $C_{1-6}$ alkyl and/or hydrogen and
$R_4$ is hydrogen or $C_{1-6}$ alkyl,
from optionally nucleus-substituted 2-benzothiazolyl disulfides, 2-mercaptobenzothiazoles or 2-mercaptobenzothiazole salts and the corresponding amines and, optionally, sulfur, which contain unreacted amine or a low-boiling alcohol used as solubilizer, or both, characterized in that the mother liquid is saturated by addition of sodium sulfate and/or sodium chloride in such a way that the pH value is adjusted to below 2 with concentrated sulfuric acid and/or concentrated hydrochloric acid, the resulting mixture is then kept at 20 to 250°C for 3 minutes to 20 hours and the pH value is then adjusted to 1 to 11 by addition of concentrated sodium hydroxide, resulting in the formation of two phases, the phases are separated from one another, the aqueous phase is extracted with more alcohol or amine at 60 to 120°C, the extractant and the extracted components are isolated from the combined organic phases and amine or alcohol dissolved in the aqueous phase is removed therefrom by distillation.

2. A process as claimed in claim 1, characterized in that, to recover the extractant, the combined organic phases are only concentrated to the extent that the residue separates into two liquid phases, a low-salt concentrate of extracted benzothiazole derivatives in the form of a melt on the one hand and an aqueous solution of the co-extracted inorganic salts on the other hand, the two phases are separated from one another and separately worked up.

3. A process as claimed in claims 1 and 2, characterized in that any N-chloramines still present in the mother liquor are destroyed by addition of reducing agents before the mother liquor is actually worked up.

4. A process as claimed in claims 1 to 3, characterized in that a pH below 1 is adjusted with concentrated acid, after which the pH is adjusted to 4 to 10 with sodium hydroxide.

5. A process as claimed in claims 1 to 4, characterized in that the mixture is kept at 60 to 120°C for 30 minutes to 2 hours between the addition of acid and sodium hydroxide.

**Revendications**

1. Procédé pour le traitement de liqueurs-mères aqueuses provenant de la fabrication par oxydation de composés de formule

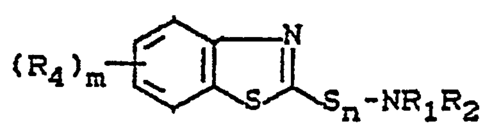

dans laquelle
m représente 0,1 ou 2,
n représente 1 ou 2,
$R_1$ représente l' hydrogène, un groupe alkyle en $C_1$ à $C_6$ ou cyclo-alkyle en $C_5$ à $C_8$,
$R_2$ représente un groupe alkyle en $C_1$ à $C_6$ ou cyclo-alkyle en $C_5$ à $C_8$ ou
$R_1$ et $R_2$ représentent ensemble $-(CH_2)_5-$ ou

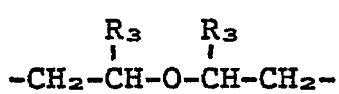

et
$R_3$ représente un groupe alkyle en $C_1$-$C_6$ et/ou l'hydrogène et
$R_4$ représente l'hydrogène ou un groupe alkyle en $C_1$-$C_6$
à partir de disulfures de 2-benzothiazolyle éventuellement substitués sur le noyau, de 2-mercaptobenzothiazoles ou de sels de 2-mercaptobenzothiazoles et leurs amines correspondantes ainsi qu'éventuellement de soufre et contenant une amine non transformée ou un alcool à bas point d'ébullition utilisé comme agent de solubilisation, ou les deux, caractérisé en ce que l'on sature la liqueur-mère par addition de sulfate de sodium et/ou de chlorure de sodium de manière à porter le pH à une valeur inférieure à 2, au moyen d'acide sulfurique concentré et/ou d'acide chlorhydrique concentré, puis qu'on maintient ce mélange durant un laps de temps de 3 minutes à 20 heures, à une température de 20 à 250°C, puis qu'on porte le pH à une valeur de 1 à 11, par addition de soude caustique concentrée, avec formation de deux phases, qu'on sépare ces phases entre elles, qu'on extrait la phase aqueuse avec un supplément d'alcool ou de l'amine à une température de 60 à 120°C, qu'on isole des phases organiques réunies l'agent

d'extraction et les composants extraits et qu'on élimine de la phase aqueuse par distillation l'amine ou l'alcool qui s'y trouve en solution.

2. Procédé selon la revendication 1, caractérisé en ce que, pour la récupération l'agent d'extraction, on ne concentre les phases organiques réunies que jusqu'à ce que le résidu se sépare en deux phases liquides, à savoir un concentré pauvre en sel de dérivés de benzothiazole extraits sous forme d'une masse fondue d'une part, et une solution aqueuse de sels inorganiques extraits en même temps d'autre part, et qu'on sépare les deux phases l'une de l'autre et qu'on les traite séparément.

3. Procédé selon les revendications 1 et 2, caractérisé en ce que, pour le traitement proprement dit, on détruit les N-chloramines éventuellement présentes dans la liqueur-mère par addition d'agents réducteurs.

4. Procédé selon les revendications 1 à 3, caractérisé en ce que l'on porte le pH à une valeur inférieure à 1 au moyen d'acide concentré, puis à une valeur de 4 à 10 au moyen de soude caustique.

5. Procédé selon les revendications 1 à 4, caractérisé en ce que, entre l'addition d'acide et l'addition de soude caustique, on laisse séjourner le mélange pour un temps de 30 minutes à 2 heures à une température de 60 à 120°C.